# EUROPÄISCHE PATENTSCHRIFT

(11) **EP 2 209 421 B1**
(45) Veröffentlichungstag und Bekanntmachung des Hinweises auf die Patenterteilung: **10.05.2017**
(21) Anmeldenummer: 08848916.6
(22) Anmeldetag: 11.11.2008
(51) Int. Cl.: A61B 5/145, A61M 1/16, A61M 1/34

(54) **VORRICHTUNG ZUM ERMITTELN WENIGSTENS EINER KENNZAHL DEN GLUKOSESTOFFWECHSEL EINES PATIENTEN BETREFFEND**
DEVICE FOR DETECTING AT LEAST ONE CHARACTERISTIC RELATING TO A PATIENT'S GLUCOSE METABOLISM
DISPOSITIF DE DÉTERMINATION D'AU MOINS UN INDICE RELATIF AU MÉTABOLISME DU GLUCOSE D'UN PATIENT

(30) Priorität: 12.11.2007 DE 102007053752
(43) Veröffentlichungstag der Anmeldung: 28.07.2010
(73) Patentinhaber: Fresenius Medical Care Deutschland GmbH, 61352 Bad Homburg (DE)
(72) Erfinder: SCHULTE, Elke, 97424 Schweinfurt (DE); MÜLLER, Carsten, 97502 Euerbach (DE)
(74) Vertreter: Bobbert & Partner Patentanwälte PartmbB
(86) Internationale Anmeldenummer: PCT/EP2008/009501
(87) Internationale Veröffentlichungsnummer: WO 2009/062661

(56) Entgegenhaltungen:
- EP-A- 1 872 812
- WO-A-2004/089440
- WO-A-2005/028001
- WO-A-2005/044088
- DE-A1- 2 853 897
- US-A1- 2006 009 727

## Beschreibung

Die vorliegende Erfindung betrifft eine Blutbehandlungsvorrichtung mit einer Vorrichtung zum Ermitteln wenigstens einer Kennzahl den Zuckerstoffwechsel eines Patienten betreffend gemäß dem Oberbegriff des Anspruchs 1.

Die vorliegende Erfindung betrifft eine Vorrichtung gemäß dem Oberbegriff des Anspruchs 1, wie sie aus der DE 28 53 897 A1 und der WO 2005/044088 A bekannt ist.

Der Glukosestoffwechsel und insbesondere die Behandlung erhöhter Blutzuckerwerte ist aufgrund der Schwere der Folgeerkrankungen erhöhter Blutzuckerwerte und nicht zuletzt auch aufgrund der damit verbundenen immensen Kosten für das Gesundheitssystem in den vergangenen Jahren zunehmend in das Zentrum der Aufmerksamkeit der Verantwortlichen gelangt. Vor dem Hintergrund der Tatsache, dass der Glukosestoffwechsel noch immer nicht vollends verstanden ist, überrascht es nicht, dass bis in die heutige Zeit hinein noch immer regelmäßig neue Studienprotokolle zur zukünftigen wissenschaftlichen Untersuchung des Glukosestoffwechsels publiziert werden, wie erst kürzlich wieder mit dem Kongressbeitrag *"*Glucose Uptake and Elimination Following an Intra-Dialytic Glucose Load" von Giessauf et al. zur XLIV ERA-EDTA, 21. bis 24. Juni 2007, in Barcelona, Spanien, geschehen.

Einen manifesten Diabetes mellitus zu diagnostizieren, ist heutzutage auf vergleichsweise einfache Weise möglich. Schwieriger ist es, auch Vorstufen des noch nicht klinisch manifesten Diabetes mellitus zu erkennen, welche mit gestörter Glukosetoleranz einhergehen.

Aus der Praxis sind Verfahren zum Bestimmen der Glukosetoleranz und/ oder der Insulinresistenz einer Person bekannt. Diese Verfahren dienen der Erkennung und Differenzierung einer gestörten Glukosehomöostase ("impaired fastening glucose"), bzw. eines Diabetes mellitus. Hierzu werden zunächst Blutparameter des Betroffenen bestimmt. Basierend auf den gemessenen Blutparameterwerten einerseits, und den gesamten Umständen, welche die gemessenen Werte mit beeinflusst haben können andererseits, kann der Arzt eine ggf. vorliegende gestörte Glukosehomöostase diagnostizieren.

Zu den Umständen, welche zum Stellen einer solchen Diagnose zwingend zu beachten sind, zählt u. a., ob der Patient bei Abnahme der Blutzuckerwerte nüchtern oder post-prandial gewesen ist, wieviel Zeit seit einer Test-Glukosegabe vergangen ist, ob der Patient Fieber hat, ob er in den vergangenen Tagen eine Mindestmenge an Kohlenhydraten zu sich genommen hat, und ob der Patient unter Medikation steht.

Im Falle einer Medikamenteneinnahme sind insbesondere solche Medikamente relevant, welche den Blutzuckerspiegel beeinflussen können. Zu diesen Medikamenten zählen bspw. Kortikoide, Kontrazeptiva oder orale Antidiabetika. Vom Arzt zu klären ist aber auch, ob bspw. anderes als körpereigenes Insulin verabreicht wurde. Eine solche - ärztlich nicht verordnete - Einnahme lässt sich regelmäßig bei Personen mit ausgeprägtem Wunsch nach Gewichtsabnahme beobachten. Schließlich zählen zu den Umständen, die vom Arzt beim Stellen der Diagnose einer pathologischen Glukosetoleranz zu bedenken sind, auch Erkrankungen, welche die Produktion oder Ausschüttung von Insulin beeinflussen.

Die bei der Durchführung eines Glukosebelastungstests bzw. -toleranztests erforderliche Blutentnahme nach einer oralen Einnahme von Glukose wird von manchen Patienten als unangenehm empfunden.

Da Diabetiker in Folge ihres Zuckerstoffwechsels regelmäßig auch Dialysepatienten sind, wird in der WO 98/19592 vorgeschlagen, die Messung des Blutglukose- bzw. Blutzuckerspiegels während einer Dialysesitzung über die Dialysevorrichtung vorzunehmen. Da zu Zwecken der Dialysebehandlung ohnehin bereits ein Zugang zu Gefäßen des Patienten gelegt wurde, lassen sich mit dem in der WO 98/19592 beschriebenen Verfahren aktuelle Blutzuckerwerte bestimmen, ohne dass hierzu eigens Blut genommen werden müsste.

Eine pathologische Glukosehomöostase, bei welcher die Blutzuckerwerte grenzwertig messbar sind, lässt sich mit dem in der WO 98/19592 beschriebenen Verfahren jedoch nicht feststellen. Zudem kann die Durchführung einer Dialyse - welche zu veränderter Zusammensetzung des zu behandelnden Bluts führen soll und führt - die Blutzuckermessung negativ beeinflussen und zu Unkorrektheiten der gemessenen Blutglukosewerte führen.

Die vorliegende Erfindung hat die Aufgabe, eine verbesserte Vorrichtung zum Untersuchen des Zuckerstoffwechsels eines Patienten und insbesondere zum Bestimmen von Kennzahlen den Blutzucker des Patienten betreffend, anzugeben.

Die erfindungsgemäße Aufgabe wird gelöst durch die Blutbehandlungsvorrichtung mit der Merkmalskombination des Anspruchs 1. Die vorliegende Erfindung ist nicht auf das hierin offenbarte Verfahren gerichtet.

Es wird ein Verfahren offenbart, mittels welchem wenigstens eine Kennzahl bzw. ein Parameterwert bzw. eine Ausprägung eines Parameters den Glukose- bzw. Zuckerstoffwechsel eines Patienten betreffend ermittelt werden kann. Das Verfahren umfasst ein extrakorporales Zugeben von Blutzucker und optional zusätzlich Insulin während einer extrakorporalen Behandlung des Blutes des Patienten, sowie ein extrakorporales Messen einer Glukosekonzentration und optional zusätzlich einer Insulinkonzentration.

Im Rahmen der vorliegenden Offenbarung wird unter einer Kennzahl zur Beschreibung des Zuckerstoffwechsels eine Blutzuckerkonzentration, eine Insulinkonzentration, ein Verlauf der zuvor genannten Konzentrationen über der Zeit sowie jede weitere Kennzahl verstanden, welche dem Fachmann einen relevanten Hinweis auf den Zuckerstoffwechsel des Patienten und insbesondere auf einen pathologischen Zustand hiervon geben kann.

Unter Zuckerstoffwechsel oder Glukosestoffwechsel wird erfindungsgemäß allgemein der Kohlenhydratstoffwechsel verstanden. Die Offenbarung soll daher weder auf die Gabe, noch auf die Messung von Glukose als Zucker beschränkt werden. Jeder andere Zucker, dessen Konzentration im Blut im Zusammenhang mit der vorliegenden Erfindung zur Aussage darüber beiträgt, ob ein Patient bspw. ein Diabetiker ist oder eine pathologische Glukosetoleranz aufweist, ist ebenfalls vom Begriff des Zucker- oder Glukosestoffwechsels, wie in dieser Patentanmeldung verwendet, umfasst. Dies gilt ungeachtet dessen, dass im Folgenden die Begriffe Glukose und Zucker synonym verwendet werden.

Unter einem extrakorporalen Zugeben von Glukose und optional zusätzlich Insulin wird erfindungsgemäß deren Zugabe in die zur extrakorporalen Behandlung des Patientenbluts verwendete Apparatur verstanden. Diese Apparatur umfasst insbesondere die arterielle(n) sowie die venöse(n) Leitung(en) eines extrakorporalen Kreislaufs. Im erfindungsgemäßen Falle einer Dialyse umfasst die Apparatur zusätzlich das Dialysat bzw. das Substituat - falls eine Substitution vorgesehen ist - sowie alle weiteren Leitungen und Bauteile der zur Dialyse - gleich ob bspw. zur Hämodialyse, Hämofiltration oder Hämodiafiltration - eingesetzten Vorrichtung.

Unter dem Begriff "extrakorporal" wird im Zusammenhang mit der Gabe von Glukose und optional zusätzlich Insulin erfindungsgemäß eine parenterale Gabe verstanden.

Im Zusammenhang mit dem Messen der Glukose- und optional zusätzlich der Insulinkonzentration wird unter dem Begriff "extrakorporal" erfindungsgemäß ein Messen verstanden, zu dessen Durchführung nicht eigens ein Zugang gelegt werden muss, sondern vielmehr ein zum Zweck der parallel durchgeführten Blutbehandlung bereits vorliegender Gefäßzugang, zu welchem erfindungsgemäß insbesondere auch ein venöses oder arterielles Leitungs- bzw. Schlauchsystem zur Blutbehandlung zählt, verwendet wird. Es wird ausdrücklich darauf hingewiesen, dass auch eine unblutige Messung bspw. durch die Haut des Patienten mittels optischer Verfahren ohne Kontakt zum Blut, und auch eine Messung einer Konzentration im Dialysat erfindungsgemäß als extrakorporal gelten.

Das Messen der Glukosekonzentration kann zu bestimmten Zeitpunkten während der extrakorporalen Behandlung des Patienten - bspw. zu Beginn einer Dialysebehandlung, zyklisch oder auf Anforderung durch den Anwender - erfolgen. Sie kann aber auch kontinuierlich erfolgen.

Wird die Glukosekonzentration und optional zusätzlich die Insulinkonzentration im extrakorporal strömenden Blut des Patienten gemessen, so handelt es sich um eine Bestimmung der Konzentration im Blut des Patienten. Unter dem Ausdruck "Messen der Glukosekonzentration und optional zusätzlich der Insulinkonzentration" wird erfindungsgemäß darüber hinaus jedoch bspw. auch ein Messen einer Menge an Glukose, welche durch einen Filter tritt, oder um welche sich die Menge eines in einem Dialysat vorliegenden Blutzuckers ändert, verstanden. Dass solche "Mengen" an Blutzucker oder Insulin stets auch als Konzentrationen ausgedrückt werden können, und dass die Erfindung somit sowohl ein Messen von Mengen als auch von Konzentrationen umfasst, versteht der Fachmann ohne weitere Erläuterung.

Das Messen der Glukosekonzentration und optional zusätzlich der Insulinkonzentration kann mittels eines oder mehrerer Sensoren im extrakorporalen Blutkreislauf erfolgen. Im Falle der Dialysebehandlung des Patienten kann zudem auch auf der Dialysatseite gemessen werden (zusätzlich zu weiteren Messungen oder ausschließlich), da Glukose die bei Dialysebehandlungen üblichen Filter passieren kann.

Bei einer Messung der Blutzuckerkonzentration und insbesondere des Blutzuckerspiegels des Patienten kommen verschiedene Verfahren in Frage. Zu ihnen zählen dem Fachmann bekannte enzymatische Verfahren, optische Verfahren wie Absorption, Polarisation oder Spektroskopie, die Messung osmotischer Änderungen, die Messung von Leitfähigkeitsänderungen sowie weitere, dem Fachmann bekannte Verfahren.

Unter "Messen" wird erfindungsgemäß auch eine extrakorporale Blutentnahme - bspw. über ein Bauteil der Vorrichtung zur Blutbehandlung - und die Zuführung der entnommenen Probe zu einem entsprechenden Labor verstanden. Das Labor kann sich dabei außer Haus befinden. Das Labor kann jedoch auch in der Vorrichtung zur Blutbehandlung vorgesehen sein, oder mit dieser am Ort der Behandlung des Patienten zusammenwirken.

Das hierin offenbarte Verfahren zeichnet sich u. a. dadurch aus, dass aufgrund der extrakorporalen und damit parenteralen Glukosegabe, und der hierdurch bewirkten Umgehung des Verdauungstraktes, ein von der Kinetik und ggf. vorliegenden Störungen der Aufnahme der Glukose durch die Verdauungsorgane unabhängiges, und somit genaueres, Erkennen einer ggf. vorliegenden gestörten Glukosetoleranz ermöglicht wird.

Durch die Nutzung der zur extrakorporalen Behandlung des Bluts bereits vorliegenden Zugänge (wobei auch ein einziger Zugang genügen würde) bedarf es dabei jedoch keines eigens gelegten intravenösen Zugangs, wie es zur Ausführung eines vorteilhafterweise nicht oralen Glukosetoleranztests an sich erforderlich ist. Das hierin offenbarte Verfahren ist somit schonender und vor allem angenehmer für den Patienten. Zudem wird dadurch, dass kein weiterer Zugang gelegt werden muss, das Infektionsrisiko vorteilhaft gesenkt.

Ein weiterer Vorteil der vorliegenden Erfindung besteht darin, dass die für den Glukosetoleranztest erforderlichen Messungen begleitend zu einer ohnehin stattfindenden Therapie ablaufen können. Der Patient braucht sich daher nicht eigens Zeit für die Durchführung des hierin offenbarten Verfahrens nehmen. Dieses läuft vielmehr während jener Zeit ab, welche der Patient zur Durchführung der extrakorporalen Blutbehandlung ohnehin in der Klinik oder Praxis oder im Fall der Heimdialyse an der Behandlungseinheit verbringt.

Da das hierin offenbarte Verfahren aber quasi nebenbei erfolgen kann, weder zusätzliche Zeit noch Mühen vom Patienten erfordert und auch keine zusätzlichen Unannehmlichkeiten mit sich bringt, ist die Hemmschwelle von manchen Patienten, ihren Glukosestoffwechsel untersuchen zu lassen, bei Anwendung des hierin offenbarten Verfahrens ungleich niedriger als mit üblichen Verfahren.

Eine gestörte Glukosetoleranz kann mittels des zum Messen von Kennzahlen eingesetzten, hierin offenbarten Verfahrens daher deutlich früher erkannt werden, als es bei der herkömmlichen Durchführung mit der hierzu erforderlichen Überzeugung des Patienten durch den behandelnden Arzt und der oft schwierigen Einbestellung des Patienten der Fall wäre. Ein frühzeitigerer Beginn der Reduzierung der zukünftigen Blutzuckeraufnahme und ein frühzeitigeres Senken des Blutzuckerspiegels in Zukunft zum Verhindern, zumindest aber zum Verzögern des Auftretens von (Folge-) Erkrankungen, welche durch erhöhten Blutzucker bekanntermaßen bedingt sind, ist aufgrund des Einsatzes des hierin offenbarten Verfahrens daher ebenfalls vorteilhaft möglich.

Des Weiteren erlaubt das hierin offenbarte Verfahren bei bilanzierter Variation der Blutglukosekonzentration oder der extrakorporal zugegebenen Menge an Glukose die Messung bzw. Bestimmung des aktuellen Blutvolumens sowie eine vereinfachte Rezirkulationsmessung, was regelmäßig zum Zwecke einer Kontrolle der korrekten Durchführung der extrakorporalen Blutbehandlung von Interesse ist.

Es wird darauf hingewiesen, dass das hierin offenbarte Verfahren vorteilhaft auch zur Untersuchung endokriner Störungen, welche mit einer pathologischen Sekretion von Insulin einhergehen, wie oben beschrieben eingesetzt werden kann.

Vorteilhafte Weiterbildungen der vorliegenden Erfindung sind jeweils Gegenstand der Unteransprüche.

Es wird in einer bevorzugten Ausführungsform vorgeschlagen, dass die Vorrichtung konfiguriert ist, die Konzentration des Zuckers bzw. der Glukose im Dialysat, also die Glukose, welche üblicherweise einer Dialysatflüssigkeit zur Behandlung des Patienten beim Zusammensetzen derselben zugesetzt wird, basierend auf der gemessenen Glukosekonzentration, und insbesondere basierend auf einem gemessenen Blutzuckerspiegel, zu verändern. Diese Veränderung kann kontinuierlich oder zu konkreten Zeitpunkten erfolgen.

Mittels dieser Veränderung kann, wenn sie im Sinne einer Angleichung der Glukosekonzentration des Dialysats an jene des Bluts erfolgt, vorteilhaft die Blutzuckerverschiebung über den Dialysefilter minimiert oder gar verhindert werden. Deshalb kann ein Blutzuckeranstieg nach der extrakorporalen Gabe von Glukose korrekt bestimmt werden, und zwar ohne dass das Messergebnis durch die Verschiebung von Glukose aus dem Blut durch den Dialysefilter hindurch in das Dialysat verfälscht werden würde. Die Dialysatglukosekonzentration wird der Blutglukosekonzentration, mit anderen Worten ausgedrückt, nachgefahren.

Nach dem Belastungstest - bei Bedarf aber auch schon während dessen Durchführung - kann eine entsprechende Angleichung, d. h. eine Veränderung im Sinne einer Absenkung, jedoch auch eine rasche Rückkehr des Blutzuckerspiegels auf einen Normwert nach erfolgter Gabe von Glukose bewirken. Zudem kann vorteilhaft sichergestellt werden, dass ein vorgegebener, maximaler Blutzuckerwert gar nicht erst erreicht werden kann. Eine Hyperglykämie wird hierdurch erfindungsgemäß vorteilhaft vermieden.

Die wenigstens eine Kennzahl kann dabei unter Berücksichtigung der Veränderung der Dialysatglukosekonzentration zur Vermeidung von Glukoseverschiebung über einen Filter - bzw. basierend hierauf - ermittelt werden, wie in einer wiederum weiter bevorzugten Ausführungsform der Erfindung vorgesehen ist. Auf diese Weise kann der Vorgang des extrakorporalen Messens des Blutzuckerspiegels zumindest teilweise unterbleiben bzw. vorteilhaft vereinfacht werden.

In einer wiederum weiter bevorzugten Ausführungsform der Erfindung ist es vorgesehen, eine Blutzuckerverschiebung über einen Dialysefilter nach dem extrakorporalen Zugeben von Blutzucker zuzulassen und ein Maß für die erfolgte Verschiebung zu ermitteln. Die wenigstens eine Kennzahl kann dabei unter Berücksichtigung der Glukoseverschiebung über die Filtereinrichtung - bzw. basierend hierauf - ermittelt werden.

Dieses Vorgehen zeichnet sich vorteilhaft dadurch aus, dass zum einen eine genauere und korrektere Ermittlung der gewünschten Kennzahl erfolgen kann, da eine dieser Bestimmung abträgliche Blutzuckerverschiebung rechnerisch kompensiert bzw. berücksichtigt wird. Zudem zeichnet sich diese erfindungsgemäße Ausführungsform auch dadurch aus, dass es keine vorrichtungsseitige Angleichung der Dialysatkonzentration erfordert, wie dies in anderen Ausführungsformen beschrieben ist. Es genügt vielmehr, eine rechnerische Berücksichtigung bzw. "Korrektur" der gemessenen Glukosekonzentration anhand der bekannten Glukoseverschiebung über den Filter bei der Ermittlung der Kennzahl vorzunehmen.

Letzteres führt zu einer apparativen Vereinfachung und damit zur Senkung der erforderlichen Herstellkosten für eine Vorrichtung zur Ausführung der Erfindung. Es führt weiter zu verringertem Wartungs- und Instandhaltungsaufwand unter Verringerung der hiermit verbundenen Kosten und des erforderlichen Zeitaufwandes.

Eine wiederum weiter bevorzugte erfindungsgemäße Ausführungsform sieht eine Ausgestaltung der Vorrichtung zum extrakorporalen Zugeben von Zucker und optional zusätzlich Insulin auf Dialysatseite oder auf Substituatseite - falls ein Substituat vorgesehen ist - vor. Die Zugabe von Zucker und optional zusätzlich Insulin kann hierbei teilweise oder vollständig auf der Dialysat- und/ oder auf der Substituatseite erfolgen. Dies erfordert einen besonders geringen technischen Aufwand, ermöglicht eine insgesamt vereinfachte Konstruktion und trägt damit zum Einsparen von Kosten bei. Von Vorteil ist hierbei ebenfalls, dass eine Zugabestelle auf der arteriellen oder venösen Seite nicht erforderlich ist. Eine Verbindung mit den extrakorporalen Blutkreislauf zur Zugabe von Glukose und/oder Insulin kann daher vorteilhaft entfallen. Dies trägt zur Vermeidung von Infektionen und Leckagen aus dem Blutkreislauf bei. Die Zugabe von Glukose und optional zusätzlich Insulin auf der Dialysatseite erlaubt ferner vorteilhaft eine genauere Steuerung der Zugabe. Die Zugabe kann zudem kontinuierlicher erfolgen, als dies - insbesondere bei vergleichbarem technischen Aufwand - bei Zugabe auf der Blutseite, beispielsweise mit einer Spritzenpumpe, der Fall ist. Die Zugabe kann mittels einer eigens hierzu vorgesehenen Zugabeeinrichtung erfolgen. Diese Zugabeeinrichtung kann zusätzlich zu einer Einrichtung zum Erzeugen und/oder Zugeben von Dialysat, welches zur Behandlung des Patienten eingesetzt werden, vorgesehen sein. Sie ist zur Untersuchung des Glukosestoffwechsels vorgesehen. Sie kann dabei ausdrücklich nicht auch zur Behandlung des dialysepflichtigen Patienten vorgesehen sein. Die Zugabeeinrichtung kann allerdings zur Erzielung von Synergien, zur Vermeidung von zusätzlichem technischen Aufwand usw. mit einer Einrichtung zum Herstellen und/oder Zugeben von Glukose in das Dialysat zur Behandlung des dialysepflichtigen Patienten funktionell und/oder strukturell verbunden sein. So ist die Zugabeeinrichtung zum Zwecke der Untersuchung des Glukosestoffwechsels durch eine Steuerung oder Regelung ausgeführt, welche eine Zugabe von Glukose und optional zusätzlich Insulin ermöglicht, welche sich von der Zugabe von Glukose zum Dialysat unterscheidet. Die Steuerung oder Regelung kann Teil der herkömmlichen Einrichtung zum Herstellen eines Dialysats unter Verwendung von Glukose sein. Die Zugabe von Glukose zum Untersuchen des Stoffwechsels kann in Form eines Bolus erfolgen. Sie kann auch in Form einer zumindest vorübergehend erhöhten Glukosezugabe zum Dialysat erfolgen.

Die hiermit verbundenen Vorteile umfassen insbesondere einen verringerten Arbeitsaufwand für den betreuenden Arzt, das Erzielen einer erhöhten Genauigkeit in der Durchführung des Glukosebelastungstests, bspw. durch genaues Einhalten von Zeiträumen zwischen einzelnen Schritten oder Messungen bei der Durchführung des Belastungstests. Ein weiterer Vorteil besteht in einer exakten Reproduzierbarkeit der Untersuchungsbedingungen.

In jeder der zuvor genannten Ausführungsformen der Erfindung kann diese eine Einrichtung zum Ausgeben oder Anzeigen und/ oder ein Speichern wenigstens einer ermittelten Kennzahl umfassen. Bei der Ausgabe kann die aus der Messung gewonnene Information dem Anwender grafisch (bspw. als Kurvenverlauf) oder in Form von Zahlenwerten auf Papier oder Monitor zur Verfügung gestellt werden. Es kann auch eine Übertragung der Daten auf andere Geräte (sog. "Telemonitoring") erfolgen. Die Speicherung dient u. a. dem späteren Nachweis, sowie einem Vergleich mit weiteren Testergebnissen früher oder später durchgeführter Messungen und anderen Untersuchungen. Sie kann mittels üblicher Speichermedien erfolgen.

Die vorliegende Erfindung wird anhand des folgenden Beispiels exemplarisch detaillierter erläutert.

So kann einem Patienten während einer Dialysesitzung oder jeder anderen extrakorporalen Behandlung des Bluts eine bestimmte Menge Glukose - bspw. 0,5 Gramm/Kilogramm Körpergewicht - in einem vorgegebenen Zeitfenster von bspw. 2 Minuten über den venösen Schenkel des extrakorporalen Kreislaufs zugegeben werden. Hierzu bedarf es keines eigens gelegten Zuganges, und dennoch kann der Belastungstest parenteral durchgefühlt werden mit den hierzu bekannten Vorteilen.

Aus dem Verlauf der Blutzuckerkurve können Kennzahlen zur Beurteilung der Glukosetoleranz berechnet werden. Dies kann bspw. der k-Wert sein, welcher sich aus k=ln2/T_{1/2}*100 ergibt. Dabei bezeichnet T_{1/2} die Halbwertszeit von Glukose. Ein Wert für k größer als 1,2 kann hierbei nach Abklärung aller weiteren, für den Arzt ebenfalls relevanten, eingangs genannten Einflussfaktoren ein Hinweis auf eine physiologische Glukosetoleranz sein, wohingegen ein Wert von k kleiner als 1,0 auf eine gestörte Glukosetoleranz hinweisen kann.

Alternativ kann die Zeitdauer bestimmt und später vom Arzt ausgewertet werden, welche bis zum Erreichen markanter Werte - bspw. des Basiswertes - vergeht. Dies kann bei Diabetikern mehr als 80 Minuten, beim Gesunden 40 bis 70 Minuten nach Injektion von 15 Gramm Glukose/m² Körperoberfläche (ivGTT nach Derot) ergeben. Zusätzlich zur Glukosekinetik kann der Insulinspiegel im Blut gemessen und beurteilt werden. Die Messung der Insulinkonzentration kann wahlweise maschinenseitig erfolgen, oder vom Anwender im Labor bestimmt werden. Die zur Durchführung des erfindungsgemäßen Verfahrens eingesetzte Vorrichtung kann dem Anwender nach festgelegten Zeitintervallen Signale zur Probenentnahme geben, oder selbstständig bzw. automatisch Messwerte erheben.

Es wird darauf hingewiesen, dass anstelle einer kurzzeitigen, hohen Glukosegabe auch eine lang andauernde Glukoseinfusion über das Dialysat erfolgen kann, und gleichzeitig die Glukose und optional zusätzlich Insulinwerte gemessen werden können.

Die vorliegende Erfindung wird zusätzlich anhand der Zeichnung - in welcher gleiche Bezugszeichen gleiche oder identischen Komponenten bezeichnen - detaillierter erläutert. In der Zeichnung gilt:
- Fig. 1: zeigt schematisch vereinfacht einen extrakorporalen Blutkreislauf mit Zugabestellen für Glukose und optional zusätzlich Insulin gemäß der vorliegenden Erfindung;
- Fig. 2: zeigt den Blutkreislauf der Fig. 1 mit zusätzlichen Einrichtungen.

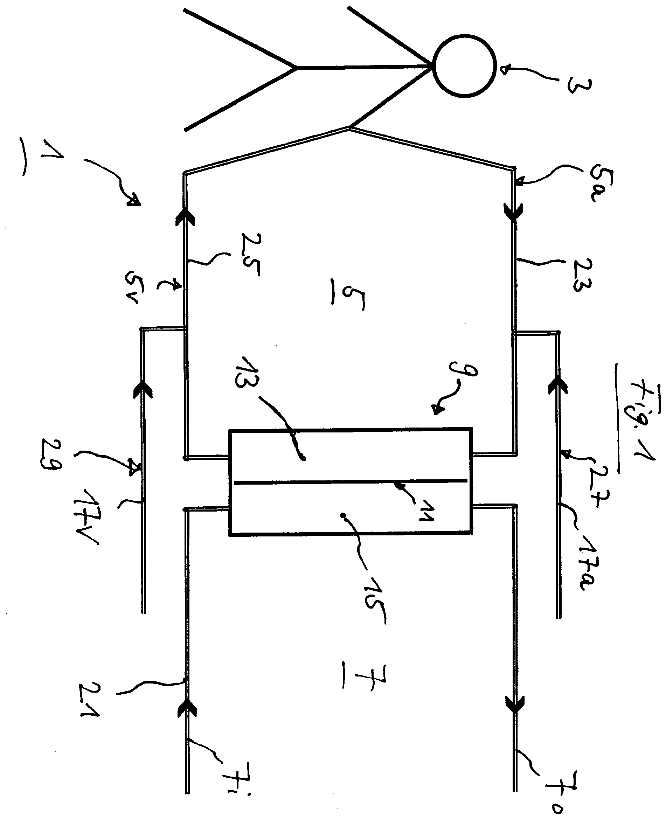

Fig. 1 zeigt schematisch vereinfacht einen extrakorporalen Blutkreislauf 1 zur Behandlung eines Patienten 3, wobei der Blutkreislauf 1 einen extrakorporalen Kreislauf 5 sowie einen unvollständig dargestellten Dialysatkreis 7 umfasst. Sowohl der extrakorporale Kreislauf 5, als auch der Dialysatkreis 7 fließen in einen Dialysator 9 hinein und wieder heraus. Der Dialysator 9 weist eine Dialysatormembran 11 auf, welche zwischen einer Blutkammer 13 und einer Dialysatkammer 15 angeordnet ist und eine Filterfunktion ausübt.

Der extrakorporale Kreislauf 5 weist einen arteriellen Schenkel 5a und einen venösen Schenkel 5v auf. Der Dialysatkreis 7 weist einen Schenkel 7i für den Dialysatzufluss und einen Schenkel 7o für den Dialysatabfluss auf.

Im Blutkreislauf 1 der Fig. 1 ist ferner ein unvollständig dargestellter Substituatzulauf bzw. eine Prädilutionsleitung 17a, welcher auf der arteriellen Seite des extrakorporalen Blutkreislaufs in den Schenkel 5a zuläuft, und ein ebenfalls unvollständig dargestellter Substituatzulauf bzw. eine Postdilutionsleitung 17v, welcher in den venösen Schenkel 5v des extrakorporalen Kreislaufs 5 mündet, vorgesehen. Die Substituatzuläufe 17a und 17v sind hierbei optional und nicht standardmäßig vorgesehen.

Die in Fig. 1 eingezeichneten Pfeile geben jeweils die Strömungsrichtung in den zugehörigen Schenkeln bzw. Leitungen an.

In Fig. 1 sind ferner fünf mögliche Punkte für eine Injektion von Glukose und/ oder Insulin eingezeichnet. Diese Punkte sind mit 21, 23, 25, 27 und 29 bezeichnet. Im Punkt 21 können die Glukose bzw. das Insulin dem Dialysat beigegeben werden, im Punkt 23 können die genannten Substanzen dem extrakorporalen Kreislauf 5 vor dem Dialysator 9, im Punkt 25 dem extrakorporalen Blutkreislauf 5 nach dem Dialysator 9 zugegeben werden. Im Punkt 27 werden die Substanzen dem Substituatzulauf auf arterieller Seite und im Punkt 29 dem Substituatzulauf auf venöser Seite zugegeben. Die Zugabe kann dabei jeweils mittels einer oder mehrerer -nicht dargestellter-Einrichtung(en) erfolgen.

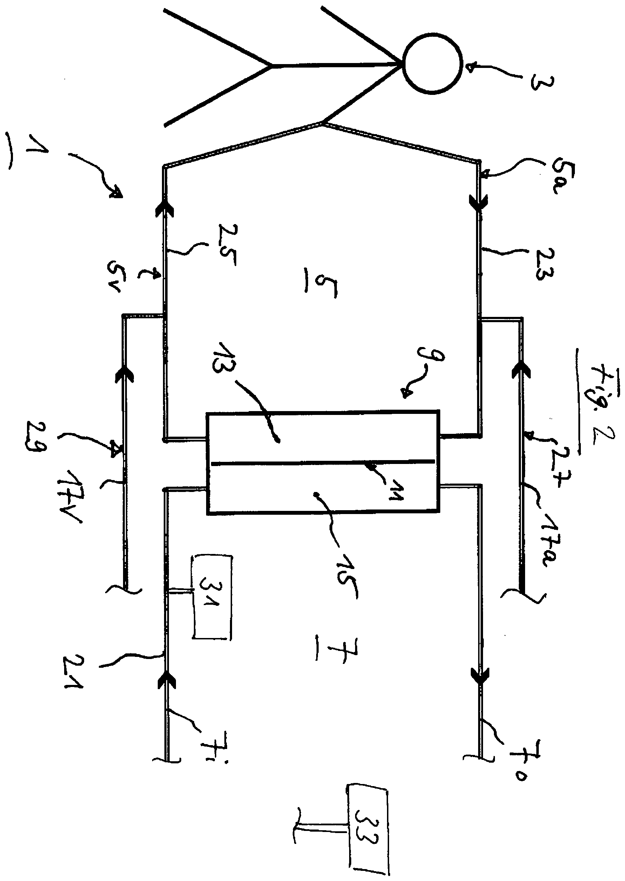

Fig. 2 zeigt den Blutkreislauf der Fig. 1. Zusätzlich ist in Fig. 2 eine Einrichtung 31 gezeigt, mittels welcher zum Zwecke der Untersuchung des Glukosestoffwechsels dem extrakorporalen Kreislauf Glukose zugegeben werden kann. Dabei ist die Zugabestelle der Fig. 2 nur eine der möglichen Zugabestellen. Weitere Stellen sind denkbar.

Fig. 2 zeigt ferner eine Einrichtung 33 zum Zugeben von Glukose zu einer Dialysatflüssigkeit bzw. einem Dialysat zur Behandlung des Patienten 3, wie sie aus dem Stand der Technik bekannt ist. Die Einrichtungen 31 und 33 können auf geeignete Weise miteinander verbunden sein. Es ist auch vorstellbar, dass eine Einrichtung die Funktionen beider Einrichtungen 31 und 33 übernimmt. Dazu muss sie auf geeignete Weise vorbereitet sein.

## Patentansprüche

1. Blutbehandlungsvorrichtung,
ausgestaltet als eine Dialysevorrichtung zur Dialysebehandlung eines Patienten (3), welche eine Substituat- und/oder eine Dialysatseite sowie einen Dialysator (9) aufweist,
mit einer Vorrichtung, konfiguriert zum Ermitteln wenigstens einer Kennzahl den Glukosestoffwechsel des Patienten (3) betreffend während einer extrakorporalen Dialysebehandlung des Patienten (3) mittels der Dialysevorrichtung, wobei die Vorrichtung ferner aufweist:
eine Einrichtung zum Messen einer Glukosekonzentration; und
eine Zugabeeinrichtung (31), welche geeignet und vorgesehen ist zum extrakorporalen Zugeben von Glukose auf der Dialysatseite (7) und/oder der Substituatseite (17a, 17v) zum Zwecke der Untersuchung des Glukosestoffwechsels,
**dadurch gekennzeichnet, dass**
die Zugabeeinrichtung mittels einer Steuerung oder Regelung ausgestaltet ist, welche konfiguriert ist zum Veranlassen der Zugabe von Glukose in Form einer zumindest vorübergehend erhöhten Glukosezugabe zum Zwecke der Untersuchung der Glukosetoleranz, und dass
die Vorrichtung zum Ermitteln der wenigstens einen Kennzahl dazu konfiguriert ist, als die wenigstens eine Kennzahl eine Kennzahl zur Beurteilung der Glukosetoleranz aus dem Verlauf der Glukosekonzentration über die Zeit zu berechnen.

2. Blutbehandlungsvorrichtung nach Anspruch 1, wobei die Vorrichtung aufweist:
eine Einrichtung, konfiguriert zum Verändern der Dialysatglukosekonzentration unter Berücksichtigung der gemessenen Glukosekonzentration.

3. Blutbehandlungsvorrichtung nach Anspruch 2, wobei die Vorrichtung aufweist:
eine Einrichtung, konfiguriert zum rechnerischen Berücksichtigen der Veränderung beim Ermitteln der wenigstens einen Kennzahl.

4. Blutbehandlungsvorrichtung nach einem der Ansprüche 1 bis 3, wobei die Vorrichtung aufweist:
eine Einrichtung, konfiguriert zum Ermitteln einer Glukoseverschiebung über eine Dialysatormembran (11) des Dialysators (9) nach dem extrakorporalen Zugeben von Glukose; und
eine Einrichtung, konfiguriert zum rechnerischen Berücksichtigen der Glukoseverschiebung über die Dialysatormembran (11) beim Ermitteln der wenigstens einen Kennzahl.

5. Blutbehandlungsvorrichtung nach einem der Ansprüche 1 bis 4, wobei die Vorrichtung aufweist:
eine Einrichtung zum Ausgeben und/ oder Speichern der wenigstens einen Kennzahl.

## Claims

1. A blood treatment apparatus,
designed as a dialysis apparatus for a treatment by dialysis of a patient (3) having a substituate side and a dialysate side as well as a dialyzer (9),
with an apparatus configured to determine at least one characteristic figure relating to the glucose metabolism of the patient (3) during an extracorporeal treatment by dialysis of the patient (3) by means of the dialysis apparatus, wherein the apparatus further comprises:
a device for measuring a glucose concentration; and
an addition device (31) intended and provided for the extracorporeal addition of glucose on the dialysate side (7) and/or on the substituate side (17a, 17v) for the purpose of analysing the glucose metabolism,
**characterized in that**
the addition device is equipped with a control or regulating device which is configured to initiate the addition of glucose in the form of an at least temporary increased addition of glucose for the purpose of analysing the glucose metabolism, and **in that**
the apparatus for determining the at least one characteristic figure is configured to calculate a characteristic figure for evaluation of the glucose tolerance from the evolution of the glucose concentration over time as the at least one characteristic figure.

2. The blood treatment apparatus according to claim 1, wherein the apparatus comprises:
a device configured to modify the glucose concentration of the dialysate in consideration of the measured glucose concentration.

3. The blood treatment apparatus according to claim 2, wherein the apparatus comprises
a device configured for arithmetical consideration of the modification during determination of the characteristic figure.

4. The blood treatment apparatus according to any one of the claims 1 to 3, wherein the apparatus comprises:
a device configured to determine a glucose shift over a dialyzer membrane (11) of the dialyzer (9) after the extracorporeal addition of glucose, and
a device configured for the arithmetical consideration of the glucose shift over the dialyzer membrane (11) during the determination of the at least one characteristic figure.

5. The blood treatment apparatus according to any one of the claims 1 to 4, wherein the apparatus comprises:
a device for outputting and/or for storing the at least one characteristic figure.

## Revendications

1. Un appareil de traitement du sang conçu comme appareil de dialyse prévu pour le traitement par dialyse d'un patient (3), comprenant une partie substitut et une partie dialysat ainsi qu'un dialyseur (9),
avec un appareil configuré pour déterminer au moins un indicateur caractéristique relatif au métabolisme glucidique du patient (3) pendant un traitement de dialyse extracorporel du patient (3) au moyen de l'appareil de dialyse, l'appareil comprenant de plus:
un dispositif prévu pour mesurer une concentration de glucose; et
un dispositif d'ajout (31) prévu et adapté pour l'ajout de glucose dans la partie dialysat (7) et/ou dans la partie substitut (17a, 17v) dans le but de l'analyse du métabolisme glucidique,
**caractérisé en ce que**
le dispositif d'ajout est équipé d'une commande ou d'une régulation, laquelle est configurée pour induire l'ajout de glucose sous la forme d'un ajout de glucose accru, tout au moins temporairement, dans le but de l'analyse de la tolérance au glucose, et **en ce que**
l'appareil prévu pour déterminer au moins un indicateur caractéristique est configuré pour calculer en tant qu'indicateur caractéristique, un indicateur caractéristique prévu pour l'évaluation de la tolérance au glucose à partir de l'évolution de la concentration de glucose relativement au temps.

2. L'appareil de traitement du sang selon la première revendication, l'appareil comprenant:
un dispositif configuré pour modifier la concentration de glucose du côté dialysat tout en tenant compte de la concentration de glucose mesurée.

3. L'appareil de traitement du sang selon la seconde revendication, l'appareil comprenant:
un dispositif configuré pour prendre en compte arithmétiquement la modification lors de la détermination d'au moins un indicateur caractéristique.

4. L'appareil de traitement du sang selon l'une quelconque des revendications 1 à 3, l'appareil comprenant:
un dispositif configuré pour déterminer un déplacement du glucose au travers d'une membrane (11) du dialyseur (9) après l'ajout extracorporel de glucose; et
un dispositif configuré pour prendre en compte arithmétiquement le déplacement du glucose au travers de la membrane (11) du dialyseur lors de la détermination d'au moins un indicateur caractéristique.

5. L'appareil de traitement du sang selon l'une quelconque des revendications 1 à 4, l'appareil comprenant:
un dispositif pour délivrer et/ou enregistrer au moins un indicateur caractéristique.
